## Europäisches Patentamt

## European Patent Office

## Office européen des brevets

(11) Publication number: **0 081 107**
**B1**

## (12) EUROPEAN PATENT SPECIFICATION

(45) Date of publication of patent specification: **10.09.86**

(21) Application number: **82110586.3**

(22) Date of filing: **16.11.82**

(51) Int. Cl.⁴: **C 12 P 13/22** // C12R1/125

(30) Priority: **27.11.81 JP 190071/81**

(43) Date of publication of application:
**15.06.83 Bulletin 83/24**

(45) Publication of the grant of the patent:
**10.09.86 Bulletin 86/37**

(84) Designated Contracting States:
**DE FR GB**

(56) References cited:
**GB-A-2 078 731**
**US-A-3 700 558**

**CHEMICAL ABSTRACTS, vol. 82, 1975, page
449, no. 56071j, Columbus, Ohio, USA**

(73) Proprietor: **AJINOMOTO CO., INC.
5-8, Kyobashi 1-chome, Chuo-ku
Tokyo 104 (JP)**

(72) Inventor: **Kurahashi, Osamu Ajinomoto
Kashimada
Ryo, 131 No. 958 Kamishada, Saiwai-ku
Kawasaki-shi Kanagawa-ken (JP)**
Inventor: **Kamada, Masahiro
No. 46-3, Tsunashimakamimachi Kohoku-ku
Yokohama-shi Kanagawa-ken (JP)**
Inventor: **Enei, Hitoshi
No. 2-30-2, Ikego
Zushi-shi Kanagawa-ken (JP)**

(74) Representative: **Schübel-Hopf, Ursula et al
Strehl, Schübel-Hopf, Schulz Patentanwälte
Widenmayerstrasse 17
D-8000 München 22 (DE)**

(54) Process for producing L-tryptophan by fermentation.

Courier Press, Leamington Spa, England.

**0 081 107**

**Description**

This invention relates to a process for producing L-tryptophan by fermentation.

It is known that L-tryptophan, which is one of the essential amino acids and an essential component in human and animal nutrition, can be synthesized by a variety of routes, some of which involve starting with β-indolylaldehyde and hippuric acid (Ber. 39, 2515, 1906) and some of which involve starting with α-ketoglutaric acid and phenyl hydrazone (US—PS 3,019,232). In addition to these chemical synthetic processes, it is known that L-tryptophan can be produced from its precursors such as an anthranilic acid, indole or 3-indole pyruvate by the action of microorganisms.

It is also known that L-tryptophan can be produced by a fermentation process in which L-tryptophan-producing mutants of the genus *Brevibacterium* are used; various L-tryptophan-producing mutants, produced by the artificial mutation of wild strains of microorganisms of the genera *Brevibacterium, Corynebacterium,* or Bacillus, are known.

Examples of such artificial mutants are mutants resistant to tryptophan analogues such as 5-methyl tryptophan (as disclosed in Japanese Published Examined Patent Application No. 18828/1973, 39517/1978); mutants resistant to tryptophan analogues and phenylalanine analogues; and mutants resistant to these analogues and requiring an L-amino acid such as L-tyrosine, L-phenylalanine, L-methionine or L-histidine for their growth (as disclosed in Japanese Published Unexamined Patent Applications Nos. 42091/1975 and 129791/1975, Japanese Published Examined Patent Application No. 19037/1976 and Agr., Biol. Chem., 39, 343 (1975)).

Recently, there has been a great demand for L-tryptophan as a feed stuff but the demand cannot be met because L-tryptophan cannot be produced at a reasonable price by any known fermentation process or chemical synthetic process even though various processes for producing L-tryptophan are known, as stated above.

According to the present invention, there is provided a process for producing L-tryptophan by fermentation, which comprises culturing aerobically in a culture medium a mutant of the genus *Bacillus* which is resistant to azaserine and tryptophan analogues and capable of producing L-tryptophan; and recovering the L-tryptophan which accumulates in the culture medium.

The process of the present invention makes it possible to produce L-tryptophan in improved yield by a fermentation process.

More particularly, the production of L-tryptophan can be increased when resistance to azaserine is imparted to a known tryptophan analogue-resistant and L-tryptophan producing mutant of the genus *Bacillus*.

The microorganisms employed in the process of the present invention are mutants which belong to the genus *Bacillus* and are resistant to azaserine and tryptophan analogue and capable of producing L-tryptophan in a greater yield.

Azaserine (also known as serine diazoacetate or o-diazoacetyl-L-serine) is one of the antibiotics produced by microorganisms of the genus *Streptomyces* having an antineoplastic activity and is known as one of the glutamine antagonists.

Tryptophan analogues, in the manner in which the term is used in connection with the present invention, are such chemicals which inhibit the growth of the microorganisms of the genus *Bacillus*, the inhibition being suppressed when L-tryptophan coexists in the medium. The tryptophan analogues include lower alkyltryptophans such as 5-methyl tryptophan and 6-methyltryptophan, halotryptophans such as 5-fluorotryptophn and 6-fluorotryptophan, tryptophan hydroxamate and 7-azatryptophan.

According to a preferred embodiment of the present invention, known characteristics useful for the production of L-tryptophan such as requirement of L-phenylalanine, L-leucine or L-methionine, and resistance to phenylalanine analogue, are imparted to the mutants used in the process of the present invention although the original mutant having such a characteristic as stated above does not produce L-tryptophan.

The representative mutant specimen of the present invention are:

Bacillus subtilis AJ 1 1 7 0 9 FERM-P 6225; BP 200;
($5\text{-F-Trp}^r$, $\text{AS}^r$)

Bacillus subtilis AJ 1 1 7 1 0 FERM-P 6226; BP 201;
($5\text{-F-Trp}^r$; $\text{Leu}^{-1}$, $\text{AS}^r$)

Bacillus subtilis AJ 1 1 7 1 6 FERM-P 6227; BP 202;
($5\text{-F-Trp}^r$, $\text{IM}^r$, $\text{AS}^r$)

Wherein the abbreviations in the parenthesis have the following meanings:—
$5\text{-F-Trp}^r$: resistance to 5-fluorotryptophan
$\text{AS}^r$, $\text{IM}^r$: resistance to azaserine and to indolmycin
$\text{Leu}^-$: requirement for L-Leucine for their growth.

The mutants identified above by FERM P numbers were originally deposited on 18th Nov. 1981 at the

2

Fermentation Research Institute, Agency of Industrial Sciences and Technology, Ministry of International Trade and Industry (FRI), 1—3, Higashi 1-Chome, Yatebe-machi, Tsukuba-gun, Ibaragi-ken 305, Japan.

The depositions were converted to depositions under the Budapest Treaty on October 25, 1982 with FRI as indicated by the above stated BP-numbers.

The mutants employed in the process of the present invention can be induced from parent microorganism strains by any conventional mutation method.

The first step of the induction is to mutate the parent strains with a suitable chemical mutagen such as N - methyl - N' - nitro - N - nitrosoguanidine and nitrous acid or with irradiation by ultraviolet light. The second step of the process is to select resistant mutants by picking up colonies of the microorganisms grown on plates of a nutrient agar medium containing an amount of azaserine which inhibits the growth of the parent strains. Thereafter, the mutants are evaluated for L-tryptophan productivity by a standard method.

Suitable parent strains from which the present mutants can be induced include mutants which are resistant to tryptophan analogues and capable of producing L-tryptophan, or wild strains, of the genus *Bacillus*.

The preferred mutants resistant to tryptophan analogue are, for example:

Bacillus subtilis FT-145 FERM-P 1 7 8 3
(5-F-Trp$^r$)

Bacillus subtilis FFL-5 FERM-P 1 7 8 6
(5-F-Trp$^r$, Leu$^-$)

Bacillus subtilis AJ 1 1 4 8 3 FERM-P 5 2 8 6
(5-F-Trp$^r$, IM$^r$)

FERM-p 1 7 8 3 and 1 7 8 6 (as disclosed in Japanese Published Examined Patent Application No. 39517/1978) were deposited on 19th December 1972, and FERM-P 5286 (as disclosed in Japanese Published Unexamined Patent Application No. 92796/1981) was deposited on 5th December, 1979.

When wild strains are used as the parent strains, resistance to tryptophan analogue is imparted to the wild strains prior to or after imparting resistance to azaserine to the wild strains.

The degree of resistance to azaserine of the mutants as stated above is shown in the following Experiment.

Experiment

Agar plates (8.5 cm in diameter) of the minimum medium of which the composition is given in the following Table 1, and further containing 100 microgram/ml azaserine were prepared.

TABLE 1:

Composition of minimum medium (pH 7.0)

| Component | Amount per 1.0 liter |
| --- | --- |
| Glucose | 5.0 g |
| Ammonium sulfate | 1.0 g |
| $KH_2PO_4$ | 8.65 g |
| $MgSO_4 . 7H_2O$ | 0.2 g |
| $FeSO_4 . 7H_2O$ | 10 mg |
| $MnSO_4 . 4H_2O$ | 10 mg |
| Sodium citrate | 0.5 g |
| L-Leucine | 100 mg |
| Agar* | 20 g |

* L-Leucine was added only when AJ 1 1 7 1 0 was used.

Each strain grown on an agar slant of the minimum medium was inoculated on the plate; the inoculum size being adjusted to $10^6$ cells per plate. Then the plate was incubated at 30°C for 2 days and the number of the colonies of the microorganisms grown on the plate was counted. The results obtained are shown in the following Table 2.

TABLE 2:

Degree of resistance

| Strain | Number of colonies per plate |
|---|---|
| FT-1 4 5 | 0 |
| AJ 1 1 7 0 9 | 500 |
| AJ 1 1 7 1 0 | more than 1000 |
| AJ 1 1 7 1 6 | more than 1000 |

The mutants of the present invention are cultured aerobically in a culture medium containing carbon sources, nitrogen sources, inorganic ions, and, when required, minor nutrients.

Suitable carbon sources include saccharides such as glucose, fructose and sucrose, and molasses and hydrolyzed starch containing these saccharides; organic acids such as acetic acid and citric acid; and alcohols.

Suitable nitrogen sources include, for example, ammonium sulphate, ammonium nitrate, gaseous ammonia and urea. As the inorganic ions, $K^+$, $Na^+$, $Ca^{++}$, $Fe^{++}$, $Mn^{++}$, $Mg^{++}$, $Zn^{++}$, $SO_4^{--}$, $Cl^-$ and $PO_4^{---}$ ions are suitably added to the culture medium when required.

Suitable minor nutrients include amino acids, vitamins, yeast extract, peptone, and hydrolyzed soy protein.

When mutants require nutrient elements such as L-amino acids, the nutrient elements are added to the culture medium.

Cultivation is carried out preferably under aerobic conditions, conveniently for from 1 to 4 days at a temperature ranging from 20 to 40°C with preferable adjustment of the pH of the culture medium to the range from 5.0 to 9.0 with an organic or inorganic acid or alkali. For this purpose, urea, $CaCO_3$, and gaseous ammonia are preferably used.

The L-tryptophan accumulated in the culture medium may be recovered by an entirely conventional recovering technique such as by means of an anion-exchanging resin.

Having generally described this invention, a further understanding can be obtained by reference to the following specific example which is provided herein for purpose of illustration only and is not intended to be limiting unless otherwise specified.

Example

Twenty ml portions of the culture medium of which the composition is given in the following Table 3 were placed in 500 ml flasks which were then heated at 110°C for 10 minutes. Thereafter, the contents of each flask were supplemented with 1.0 g $CaCO_3$ separately sterilized.

# 0 081 107

## TABLE 3

### Composition of culture medium (pH 7.0)

| Component | Amount per 1.0 liter |
|---|---|
| Glucose | 80 g |
| Ammonium chloride | 10 g |
| $KH_2PO_4$ | 1.0 g |
| KCl | 2.0 g |
| $MnSO_4 \cdot 7H_2O$ | 10 mg |
| $FeSO_4 \cdot 4H_2O$ | 10 mg |
| $MgSO_4 \cdot 7H_2O$ | 0.4 g |
| Hydrolyzed casein | 4 g |

Each strain for testing, shown in the following Table 4, which was previously cultured on an agar slant of the same culture medium, was inoculated into a batch of the culture medium, and cultured with shaking at 30°C for 96 hours.

After the cultivation, the determination of the L-tryptophan which accumulated in the culture broth was conducted according to a standard bio-assay method using *Leuconostoc mesenteroides* ATCC 8042 and the results obtained are shown in Table 4.

## TABLE 4:

### Amount of L-tryptophan accumulated

| Strain No. | L-tryptophan (mg/ml) |
|---|---|
| FT-1 4 5 (5-F-Trp$^r$) | 1.90 |
| AJ 1 1 7 0 9 (5-F-Trp$^r$, AS$^r$) | 3.5 |
| AJ 1 1 7 1 0 (5-F-Trp$^r$, Leu$^-$, AS$^r$) | 7.0 |
| AJ 1 1 7 1 6 (5-F-Trp$^r$, IM$^r$, AS$^r$) | 7.1 |

Culture broth of AJ 1 1 7 1 6 prepared in the same manner as described above was collected and centrifuged to remove microbial cells and solid $CaCO_3$. 1.0 liter of supernatant solution thus obtained was passed through a column of "Daiai on SK 104" in the acid form. By this procedure L-tryptophan was adsorbed on the resin, and it was eluted with 0.5$N$ $NH_4OH$. The elute was evaporated and cooled to a temperature low enough to crystallize L-tryptophan.

Then crude crystalline L-tryptophan was dissolved in an aliquot of 50% ethanol solution and active carbon was added to the solution. After decolourization was conducted, 4.5 g crystalline L-tryptophan were obtained from the decolourized solution.

## Claims

1. A process for producing L-tryptophan by fermentation, which comprises culturing aerobically in a usual culture medium a mutant of the genus *Bacillus* which is resistant to azaserine and tryptophan analogues and capable of producing L-tryptophan; and recovering the L-tryptophan which is accumulated in the culture medium.

5

2. A process according to claim 1, wherein the mutant belongs to the species *Bacillus* subtilis.
3. A process according to claim 1 or 2, wherein the mutant resistant to azaserine and tryptophan is:

Bacillus subtilis AJ 1 1 7 0 9 FERM-P 6225; BP 200
(5-F-Trp$^r$, AS$^r$)

Bacillus subtilis AJ 1 1 7 1 0 FERM-P 6226; BP 201
(5-F-Trp$^r$; Leu$^-$, AS$^r$)

Bacillus subtilis AJ 1 1 7 1 6 FERM-P 6227; BP 202
(5-F-Trp$^r$, IM$^r$, AS$^r$)

4. A process according to claim 1, 2 or 3 wherein the tryptophan analgoue is 5-methyltryptophan or 5-fluorotryptophan.

**Patentansprüche**

1. Verfahren zur Herstellung von L-Tryptophan durch Fermentation, bei dem eine Mutante des Genus *Bacillus,* die gegen Azaserin und Tryptophan-Analoge resistent ist und zur Bildung von L-Tryptophan befähigt ist, aerob in einem üblichen Kulturmedium gezüchtet wird und das in dem Kulturmedium angereicherte L-Tryptophan gewonnen wird.
2. Verfahren nach Anspruch 1, bei dem die Mutante der Spezies *Bacillus* subtilis angehört.
3. Verfahren nach Anspruch 1 oder 2, bei dem die gegen Azaserin und Tryptophan resistente Mutante eine der folgenden ist:

Bacillus subtilis AJ 1 1 7 0 9 FERM-P 6225; BP 200
(5-F-Trp$^r$, AS$^r$)

Bacillus subtilis AJ 1 1 7 1 0 FERM-P 6226; BP 201
(5-F-Trp$^r$, Leu$^-$, AS$^r$)

Bacillus subtilis AJ 1 1 7 1 6 FERM-P 6227; BP 202
(5-F-Trp$^r$, IM$^r$, AS$^r$)

4. Verfahren nach Anspruch 1, 2 oder 3, bei dem das Tryptophan-Analoge 5-Methyltryptophan oder 5-Fluortryptophan ist.

**Revendications**

1. Procédé pour la production de L-tryptophane par fermentation, qui consiste à cultiver en conditions aérobies dans un milieu de culture habituel un mutant du genre *Bacillus* qui est résistant à l'azasérine et aux analogues du tryptophane et capable de produire du L-tryptophane; et on récupère le L-tryptophane qui est accumulé dans le milieu de culture.
2. Procédé selon la revendication 1, dans lequel le mutant appartient à l'espèce *Bacillus* subtilis.
3. Procédé selon la revendication 1 ou 2, dans lequel le mutant résistant à l'azasérine et au tryptophane est:

Bacillus subtilis AJ 1 1 7 0 9 FERM-P 6225; BP 200
(5-F-Trp$^r$, AS$^r$)

Bacillus subtilis AJ 1 1 7 1 0 FERM-P 6226; BP 201
(5-F-Trp$^r$; Leu$^-$, AS$^r$) ou

Bacillus subtilis AJ 1 1 7 1 6 FERM-P 6227; BP 202
(5-F-Trp$^r$, IM$^r$, AS$^r$).

4. Procédé selon la revendication 1, 2 ou 3, dans lequel l'analogue du tryptophane est le 5-méthyltryptophane ou le 5-fluorotryptophane.